Europäisches Patentamt

European Patent Office

Office européen des brevets

(19)

(11) Veröffentlichungsnummer : **0 056 629**
**B1**

# EUROPÄISCHE PATENTSCHRIFT

(45) Veröffentlichungstag der Patentschrift :
15.05.85

(21) Anmeldenummer : 82100257.3

(22) Anmeldetag : 15.01.82

(51) Int. Cl.⁴ : **C 07 K  3/18**, A 61 K 35/16,
A 61 K 37/02, A 61 K 37/475

(54) Verfahren zur Reinigung der Blutgerinnungsfaktoren II, VII, IX und/oder X und danach hergestellte Präparationen.

(30) Priorität : 21.01.81 DE 3101752

(43) Veröffentlichungstag der Anmeldung :
28.07.82 Patentblatt 82/30

(45) Bekanntmachung des Hinweises auf die Patenterteilung : 15.05.85 Patentblatt 85/20

(84) Benannte Vertragsstaaten :
AT BE CH DE FR GB IT LI LU NL SE

(56) Entgegenhaltungen :
EP-A- 0 014 333
DE-A- 2 734 821
Chemical Abstracts Band 67, Nr. 25, 18. Dezember
1967 Columbus, Ohio, USA D. VOSS "Prothrombin
complex. I. Behavior of prothrombin and Factors VII,
IX, and X adsorbed on barium sulfate" Seite 10716,
Spalte 1, Abstract Nr. 113723q
Chemical Abstracts Band 71, Nr. 25, 22. Dezember
1969 Columbus, Ohio, USA Y. MASAKI "Purification
of prothrombin" Seiten 79 bis 80, Spalten 2, 1,
Abstract Nr. 120342a
Chemical Abstracts Band 73, Nr. 13, 28. September
1970 Columbus, Ohio, USA M.J.P. KAHN et al. "Blood
coagulation factor V. I. Interaction of salts of fatty
acids and coagulation factors" Seite 130, Spalten 1,
2, Abstract Nr. 63994e

(73) Patentinhaber : BEHRINGWERKE Aktiengesellschaft
Postfach 1140
D-3550 Marburg 1 (DE)

(72) Erfinder : Schwinn, Horst, Dr.
Stümpelstal 27
D-3550 Marburg/Lahn (DE)
Erfinder : Heimburger, Norbert, Prof. Dr.
Sonnenhang 10
D-3550 Marburg/Lahn (DE)
Erfinder : Kumpe, Gerhardt
Perbaler Weg 11
D-3552 Wetter (DE)
Erfinder : Drescher, Heinz Hermann
Hindenburgstrasse 77
D-3577 Neustadt (DE)

(74) Vertreter : Meyer-Dulheuer, Karl-Hermann, Dr. et al
HOECHST Aktiengesellschaft Zentrale Patentabteilung Postfach 80 03 20
D-6230 Frankfurt/Main 80 (DE)

Chemical Abstracts Band 95, Nr. 3, 1981 Columbus, Ohio, USA E.J. McKAY "A simple two-step procedure for the isolation of antithrombin III from biological fluids" Seite 275, Spalten 1,2; Abstract Nr. 20365n
Chemical Abstracts Band 70, Nr. 11, 17. März 1969 Columbus, Ohio, USA J. SOULIER et al. "Different molecular states of Factor II(prothrombin) with sta-phylocoagulase and anti-Factor II antibody. III. Modification of Factor II (human prothrombin) in concentrated sodium citrate and ammonium sulfate solutions" Seite 168, Spalte 2, Abstract Nr. 45746j D. VOSS, Hoppe-Seyler's Z. Physiol. Chem. Bd. 348 (1967), S. 1163-1171
J.P. Kahn et al., Thromb. Diath. Haemorrh, 22(3), S. 417-30 (1969)

**Beschreibung**

Die Erfindung betrifft ein Verfahren zur Reinigung von die Blutgerinnungsfaktoren II, VII, IX und/oder X enthaltenden Flüssigkeiten durch Adsorption an an sich hierfür beschriebene mineralische Adsorbentien wie beispielsweise Calciumphosphat, Aluminiumhydroxid-Gel, Bariumsulfat oder Hydroxylapatit in Gegenwart von Calciumionen und Elution. Es ist bekannt, Blutgerinnungsfaktoren durch Adsorption an Anionenaustauscher sowie Calciumphosphat und Elution, beispielsweise aus Plasma, anzureichern und zu reinigen (Soulier, J. P. et al.: Thromb. Diath. Haemorrh. Suppl. *35*, 61 1969) ; Bruning, P. F. et al.: Hemophilia and New Hemorrhagic States, Univ. North Carolina Press, Chapel Hill, S. 3 (1979) ; Bidwell, E. et al.: Br. J. Haematol. *22*, 469 (1972).

Aus Hoppe-Seyler's Z. Physiol. Chem., 348 (1967), 1163-1171, besonders Seite 1170, geht hervor, daß Calcium in der Lage ist, die Adsorption von Gerinnungsfaktoren an Bariumsulfat zu hemmen.

Für die Therapie von Gerinnungsstörungen des Blutes werden jedoch Präparationen von Gerinnungsfaktoren von höherer Reinheit gebraucht.

Es bestand daher das Bedürfnis, ein Verfahren zu finden, welches es gestattet, Präparationen der Blutgerinnungsfaktoren II, VII, IX und/oder X aus, gegebenenfalls zur Abtötung von Hepatitisviren erhitzten, Lösungen von der heutzutage geforderten Reinheit zu gewinnen.

Diese Aufgabe wird dadurch gelöst, daß diese Blutgerinnungsfaktoren aus diese Faktoren enthaltenden Lösungen in Gegenwart von Calciumionen an an sich hierfür beschriebenen mineralischen Adsorbentien wie beispielsweise Calciumphosphat, Aluminiumhydroxid-Gel, Bariumsulfat oder Hydroxylapatit adsorbiert und eluiert werden.

Gegenstand der Erfindung ist demnach ein Verfahren zur Reinigung eines der Blutgerinnungsfaktoren II, VII, IX und/oder X durch Adsorption aus einer Lösung in Gegenwart von Calciumionen an ein mineralisches Adsorbens, dadurch gekennzeichnet, daß bei einer Konzentration der Calciumionen von 0,05 bis 2 mol/l, einer Konzentration des Adsorbens von 0,2 bis 20 % (w/v) und einem pH-Wert von 6 bis 9 adsorbiert, Adsorbens und Flüssigkeit getrennt, gegebenenfalls das Adsorbens gewaschen und der Blutgerinnungsfaktor eluiert wird.

Eine Proteinlösung, die den Blutgerinnungsfaktor in einer Konzentration von mindestens 0,05 bis 0,1 Einheiten/mg Protein enthält, wird mit einem Calciumsalz in einer Konzentration von 0,05 bis 2,0 mol/l, vorzugsweise 0,4 bis 0,6 mol/l Calciumchlorid ($CaCl_2$) und einem oberflächenaktiven Adsorbens wie Calciumphosphat, Aluminiumhydroxid-Gel, Hydroxylapatit oder Bariumsulfat in einer Konzentration von 0,2 bis 20 % w/v, vorzugsweise 0,4 bis 1 % w/v Calciumphosphat versetzt und bei einem pH-Wert von 6,0 bis 9,0 gerührt. Das Adsorptionsmittel wird von der Flüssigkeit abgetrennt, gegebenenfalls gewaschen und eluiert, wie bei Soulier J. P. et al., Thromb. Diath. Haemorrh. Suppl. 35, 61 (1969) beschrieben.

Zum Erzielen einer besseren Ausbeute muß die Proteinlösung gegebenenfalls vor der Adsorption mit Fällungsmitteln, die dem Fachmann bekannt sind, fraktioniert werden, wobei die zu wählenden Fällungsmittel die Konzentration der Calciumionen nicht verändern sollen.

Das erfindungsgemäße Verfahren ist durch den Zusatz der Calciumionen während der Adsorption den vorzitierten, literaturbekannten Verfahren überlegen. Die hohe Affinität der Blutgerinnungsfaktoren zu den genannten Adsorbentien in Gegenwart von Calciumionen wird am Beispiel von 0,5 mol/l $CaCl_2$, Calciumphosphat und dem Gerinnungsfaktor II (Prothrombin) in folgender Tabelle gezeigt :

| Calciumphosphat-Konzentration % w/v | Verbleibender F II im Überstand in % der Ausgangsaktivität | |
|---|---|---|
| | ohne $CaCl_2$ | mit $CaCl_2$ |
| 0 | 100 | 100 |
| 0,25 | 100 | 50 |
| 0,5 | 100 | 23 |
| 1,0 | 46 | 0 |
| 2,0 | 14 | — |
| 3,0 | 8 | — |

Die Gegenwart von $CaCl_2$ reduziert die zur quantitativen Adsorption notwendige Calciumphosphat-Konzentration um mehr als das Dreifache. Unspezifische Adsorptionen werden dadurch nahezu ausgeschlossen, so daß die Blutgerinnungsfaktoren nachfolgend in sehr reiner Form eluiert werden können.

Die Kontrolle der Maßnahmen zur Anreicherung und Reinigung der an Calciumphosphat, Aluminiumhydroxid-Gel, Bariumsulfat oder Hydroxylapatit adsorbierten Blutgerinnungsfaktoren sind dem Fachmann durch die Kenntnis von Bestimmungsmethoden für die betreffenden Substanzen geläufig.

Für Faktor II kann sie zum Beispiel nach der Methode von Koller, F. et al., Dtsch. med. Wochenschr. *81*, 516 (1956), erfolgen. Dazu wird ein Teil, zum Beispiel 0,1 ml Faktor-II-Mangelplasma, und ein Teil verdünntes Normalplasma vermischt. Diese Mischung wird 30 Sekunden bei + 37 °C gehalten. Danach setzt man zwei Teile calciumhaltiges Thromboplastin, z. B. hergestellt nach dem deutschen Patent 23 56 493, zu und bestimmt die Zeit, die bis zum Auftreten eines Gerinnsels verstreicht. Zur quantitativen Aussage wird die aus der Faktor II enthaltenden Lösung sich ergebende Gerinnungszeit unter Bezugnahme auf eine mit einer Normalplasma-Verdünnungsreihe erzielten Eichkurve abgelesen.

Eine Einheit Faktor II entspricht der Faktor II-Aktivität von 1 ml Normalplasma.

Der Faktor VII kann zum Beispiel nach der Methode von Koller, F. et al., Acta haemat. 6, 1 (1951) bestimmt werden. Dazu wird ein Teil, z. B. 0,1 ml Faktor VII-Mangelplasma, und ein Teil verdünntes Normalplasma vermischt. Diese Mischung wird 30 Sekunden bei + 37 °C gehalten. Danach setzt man 2 Teile calciumhaltiges Thromboplastin, z. B. hergestellt nach dem deutschen Patent 23 56 493, zu und bestimmt die Zeit, die bis zum Auftreten eines Gerinnsels verstreicht. Zur quantitativen Aussage wird die aus der Faktor VII enthaltenden Lösung sich ergebende Gerinnungszeit unter Bezugnahme auf eine mit einer Normalplasma-Verdünnungsreihe erzielten Eichkurve abgelesen.

Eine Einheit Faktor VII entspricht der Faktor VII-Aktivität von 1 ml Normalplasma.

Die Bestimmung des Faktors IX erfolgt beispielsweise nach folgendem Verfahren :

1 Teil, z. B. 0,1 ml partielles Thromboplastin, z. B. hergestellt nach der DE-AS 23 16 430, wird mit einem Teil Faktor IX-Mangelplasma und einem Teil verdünntem Normalplasma vermischt. Diese Mischung wird 6 Minuten bei 37 °C gehalten. Nach Zusatz von einem Teil einer auf 37 °C vorgewärmten 0,025 molaren Calciumchlorid-Lösung wird die Zeit bestimmt, die vom Zusatz der Calciumchlorid-Lösung bis zum Auftreten eines Gerinnsels verstreicht. Zur quantitativen Aussage wird die aus der Faktor IX enthaltenden Lösung sich ergebende Gerinnungszeit unter Bezugnahme auf eine mit einer Normalplasma-Verdünnungsreihe erzielten Eichkurve abgelesen.

1 Internationale Einheit (= 1 IE) Faktor IX entspricht der Faktor IX-Aktivität von 1 ml Normalplasma.

Der Faktor X kann beispielsweise nach der Methode von Duckert, F. et al., Blood Coagulation, Hemorrhage and Thrombosis, Ed. Tocantins, L. M. and Kazal, L. A. (1964), erfolgen. Dazu wird ein Teil, z. B. 0,1 ml, Faktor X-Mangelplasma und ein Teil verdünntes Normalplasma vermischt. Diese Mischung wird 30 Sekunden bei + 37 °C gehalten. Danach setzt man zwei Teile calciumhaltiges Thromboplastin, z. B. hergestellt nach dem DE-Patent 23 56 493, zu und bestimmt die Zeit, die bis zum Auftreten eines Gerinnsels verstreicht. Zur quantitativen Aussage wird die aus der Faktor X enthaltenden Lösung sich ergebende Gerinnungszeit unter Bezugnahme auf eine mit einer Normalplasma-Verdünnungsreihe erzielten Eichkurve abgelesen.

Eine Einheit Faktor X entspricht der Faktor X-Aktivität von 1 ml Normalplasma.

Unter Verwendung dieser Kontrollmethoden können die Verfahrensbedingungen unter dem Gesichtspunkt einer befriedigenden Ausbeute und einer befriedigenden Reinheit eines gegebenenfalls herzustellenden, die Blutgerinnungsfaktoren enthaltenden Konzentrates gelenkt werden.

Unter den erfindungsgemäßen Bedingungen erhält man bei vergleichbarer Ausbeute ein wesentlich reineres Produkt als mit den vorzitierten Verfahren, wie folgende Tabelle zeigt :

| | Herstellung der Blutgerinnungsfaktoren | | | | | | | |
| | II | | VII | | IX | | X | |
| Verfahren | 1) | 2) | 1) | 2) | 1) | 2) | 1) | 2) |
|---|---|---|---|---|---|---|---|---|
| Reinheit (spez. Akt.) Einheiten*/mg | 0,6 | 2,0 | 0,3 | 1,0 | 0,5 | 2,5 | 0,5 | 2,5 |

* 1 Einheit entspricht der Aktivität des Gerinnungsfaktors in einem ml humanen Normal-Citratplasma.
1) gemäß Soulier, J. P. et al.
2) gemäß Beispiel 1 dieser Anmeldung

Die Erfindung soll an den nachstehenden Beispielen näher erläutert werden :

Beispiel 1

Herstellung eines Faktor II, VII, IX, X-Konzentrates aus humanem Plasma.

Nach Soulier, J. P. et al. werden 7,5 Liter einer Lösung hergestellt, in der die Faktoren II, VII, IX und X von 500 Litern Human-Plasma in Gegenwart von 1 mol/l NaCl bei pH 8 enthalten sind. Die spezifische Aktivität der Faktoren II, IX und X muß jeweils $\geq$ 0,1 Einheiten/mg, des Faktors VII $\geq$ 0,05 Einheiten betragen.

Die Lösung wird auf eine Ammoniumsulfat-Konzentration von 40 % w/v gebracht. Der Niederschlag wird abzentrifugiert und verworfen. Der Überstand wird durch Dialyse von Sulfationen befreit und mit 0,25 kg $CaCl_2 \cdot 2H_2O$ und 0,25 kg $Ca_3(PO_4)_2$ versetzt und bei pH 7,6 30 Minuten gerührt. Anstelle von 0,25 kg

$Ca_3 (PO_4)_2$ können mit vergleichbarem Ergebnis 1,3 l einer 1 %igen w/v $Al(OH)_3$-Suspension eingesetzt werden.

Nach Zentrifugation wird der Überstand verworfen und das Adsorbens mit je 10 Liter NaCl-Lösung, 0,5 ml/l, zweimal gewaschen. Das Adsorbens wird mit 1,8 Liter Puffer von pH 8,0 eluiert, der 0,2 mol/l Tri-Natrium-Citrat, 0,15 mol/l NaCl, 2 g/100 ml Glycin, 0,3 E/ml Antithrombin III und 14 IE/ml Heparin enthält. Nach Zusatz von 0,2 g/100 ml kolloidaler Kieselsäure als Zentrifugationshilfsmittel wird das Eluat durch Zentrifugation bei 30 000 × g vom Adsorbens abgetrennt. Der Rückstand wird verworfen und der Überstand gegen 100 Liter eines Puffers von pH 7 mit 0,06 mol/l NaCl, 0,02 mol/l Tri-Natrium-Citrat und 2 g/100 ml Glycin drei Stunden dialysiert. Das Dialysat wird auf Faktor II, VII, IX und X-Aktivität getestet, auf die gewünschte Konzentration eingestellt, sterilfiltriert, dosiert und lyophilisiert.

Aus 500 Liter Plasma erhält man ca. 650 Abfüllungen zu 160 E Faktor II, 80 E Faktor VII, 200 E Faktor IX und 140 E Faktor X.

## Patentanspruch

Verfahren zur Reinigung eines der Blutgerinnungsfaktoren II, VII, IX oder X durch Adsorption aus einer Lösung in Gegenwart von Calciumionen an ein mineralisches Adsorbens, dadurch gekennzeichnet, daß bei einer Konzentration der Calciumionen von 0,05 bis 2 mol/l, einer Konzentration des Adsorbens von 0,2 bis 20 % (w/v) und einem pH-Wert von 6 bis 9 adsorbiert, Adsorbens und Flüssigkeit getrennt, gegebenenfalls das Adsorbens gewaschen und der Blutgerinnungsfaktor eluiert wird.

## Claim

A process for purifying one of the blood clotting factors II, VII, IX or X by adsorption from a solution on a mineral adsorbent in the presence of calcium ions which comprises adsorbing at a concentration of calcium ions from 0.05 to 2 mol/l, a concentration of the adsorbent from 0.2 to 20 % w/v and a pH from 6 to 9, separating the adsorbent and the liquid from one another, optionally washing the adsorbent and eluting the blood clotting factor.

## Revendication

Procédé de purification de l'un des facteurs de coagulation du sang II, VII, IX ou X par adsorption sur un adsorbant minéral à partir d'une solution en présence d'ions calcium, caractérisé en ce que l'on réalise l'adsorption à une concentration des ions calcium de 0,05 à 2 mole/l, à une concentration de l'adsorbant de 0,2 à 20 % (p/v) et à un pH de 6 à 9, on sépare l'adsorbant et le liquide, éventuellement on lave l'adsorbant et on élue le facteur de coagulation du sang.